(19) 

Europäisches
Patentamt
European
Patent Office
Office européen
des brevets

(11) **EP 3 888 645 A1**

(12) **EUROPEAN PATENT APPLICATION**
published in accordance with Art. 153(4) EPC

(43) Date of publication:
06.10.2021 Bulletin 2021/40

(21) Application number: 19890813.9

(22) Date of filing: 26.11.2019

(51) Int Cl.:
*A61K 31/216* (2006.01)     *A61P 29/00* (2006.01)
*A61P 37/08* (2006.01)     *A61K 36/535* (2006.01)
*A23L 19/00* (2016.01)     *A23L 33/105* (2016.01)

(86) International application number:
**PCT/JP2019/046059**

(87) International publication number:
**WO 2020/111021 (04.06.2020 Gazette 2020/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 27.11.2018 JP 2018221098

(71) Applicant: Kao Corporation
Chuo-ku,
Tokyo 103-8210 (JP)

(72) Inventors:
• **TAKAMURA, Akihiro**
 **Haga-gun, Tochigi 321-3497 (JP)**
• **IWASHITA, Masazumi**
 **Haga-gun, Tochigi 321-3497 (JP)**
• **YAMAMOTO, Yuta**
 **Kamisu-shi, Ibaraki 314-0103 (JP)**
• **YAMAWAKI, Kenji**
 **Kamisu-shi, Ibaraki 314-0103 (JP)**
• **HASHIZUME, Kohjiro**
 **Haga-gun, Tochigi 321-3497 (JP)**

(74) Representative: **Vossius & Partner
Patentanwälte Rechtsanwälte mbB
Siebertstraße 3
81675 München (DE)**

(54) **METHOD FOR PRODUCING EXTRACT OF RED SHISO LEAVES**

(57) Provided is a method for producing a red perilla leaf extract which enables to collect rosmarinic acid with a high yield from red perilla leaves to which an acid is added. The method for producing a red perilla leaf extract includes the following step (1): (1) a step of extracting red perilla leaves to which an acid has been added with water under a condition of a pH of from 4.0 to 8.0.

EP 3 888 645 A1

## Description

Field of the Invention

The present invention relates to a method for producing a red perilla leaf extract and a method for producing a purified red perilla leaf extract.

Background of the Invention

Red perilla (Perilla frutescens var. crispa f. purpurea) is an annual plant of Lamiaceae and is a plant that has been used for food and medicinal purposes since ancient times. As a medicinal ingredient of red perilla, rosmarinic acid that is mainly contained in leaves is known, and it has been reported that rosmarinic acid has an antiallergic action, an anti-inflammatory action, etc.

(Non Patent Literature 1).

Various methods for producing an extract containing rosmarinic acid from red perilla leaves have hitherto been proposed. For example, a method for producing a Lamiaceae plant extract containing rosmarinic acid by extracting raw whole leaves of red perilla with water at 50°C to 93°C under acidic conditions of a pH of from 0.5 to 4.0 (Patent Literature 1), a method for producing an extract containing phenols by extracting salted red perilla leaves with water under a condition of a pH of from 0.5 to 2.3 and under a heating condition of from 80°C to 93°C and subjecting the resulting extract to chromatography using an adsorption resin (Patent Literature 2), and a method for producing a perilla extract solution by extracting a harvested raw or sun-dried Lamiaceae plant with lower alcohol, removing the precipitate generated by addition of water, concentrating the resulting extract solution, then adding lower alcohol thereto to give an alcohol concentration of 90 vol% or more, and removing the resulting precipitate to obtain a liquid component (Patent Literature 3).
Patent Literature 1: WO 2002/62365
Patent Literature 2: JP-A-2003-180286
Patent Literature 3: JP-A-07-187989

Non Patent Literature 1: KIDO Hirotsugu, Oleo Science, 2004, 4(10), pp. 409-415

Summary of Invention

The present invention relates to the following 1) and 2):

1) A method for producing a red perilla leaf extract, comprising the following step (1):

(1) a step of extracting red perilla leaves to which an acid has been added with water under a condition of a pH of from 4.0 to 8.0; and

2) A method for producing a purified red perilla leaf extract, comprising the following steps (1) to (3):

(1) a step of extracting red perilla leaves to which an acid has been added with water under a condition of a pH of from 4.0 to 8.0;
(2) a step of concentrating the extract solution obtained in the step (1); and
(3) a step of adding ethanol or an ethanol aqueous solution to the concentrated extract solution obtained in the step (2) and then removing a precipitate.

Detailed Description of the Invention

However, in the above-described technology, rosmarinic acid is not sufficiently extracted from red perilla leaves to reduce the collection rate in some cases.
In addition, red perilla is a plant that can be harvested only in summer, and salted products prepared by impregnating red perilla with acid such as plum vinegar and table salt are broadly distributed in consideration of enhancing the preservability of red perilla leaves. Furthermore, red perilla leaves are often preserved by adding acids in consideration of causing the pigment contained in the red perilla leaves to develop a red color. Accordingly, if red perilla leaves to

which an acid has been added, not raw leaves, can be used as a raw material for extraction of rosmarinic acid, it is advantageous from the industrial viewpoint and the economic viewpoint.

Accordingly, the present invention relates to a provision of a method for producing a red perilla leaf extract, the method capable of collecting rosmarinic acid with a high yield from red perilla leaves to which an acid has been added.

The present inventors found that rosmarinic acid can be collected with a high yield by extracting red perilla leaves to which an acid has been added with water within a specific pH range. In addition, the present inventors further found that the purity of rosmarinic acid can be increased while leaving rosmarinic acid by concentrating this water extract, then adding an ethanol aqueous solution thereto, and removing the precipitated precipitate.

According to the present invention, rosmarinic acid can be collected with a high yield from red perilla leaves to which an acid has been added. Consequently, a red perilla leaf extract containing a large amount of rosmarinic acid is obtained. Furthermore, a purified red perilla leaf extract having an increased purity of rosmarinic acid while leaving rosmarinic acid can be obtained by performing purification by ethanol precipitation after extraction.

The method for producing a red perilla leaf extract of the present invention includes (1) a step of extracting red perilla leaves to which an acid has been added with water under a condition of a pH of from 4.0 to 8.0.

In addition, the method for producing a purified red perilla leaf extract of the present invention includes (1) a step of extracting red perilla leaves to which an acid has been added with water under a condition of a pH of from 4.0 to 8.0, (2) a step of concentrating the extract solution obtained in the step (1), and (3) a step of adding ethanol or an ethanol aqueous solution to the concentrated extract solution obtained in the step (2) and then removing a precipitate.

In the present invention, the term "red perilla leaf" refers to a leaf of red perilla (Perilla frutescens var. crispa f. purpurea) belonging to the genus Perilla in the family Lamiaceae. The red perilla leaves to which an acid has been added are those obtained by adding an acid to the raw red perilla leaves and may be used in a raw state or may be used as a dried form or a salted form.

Examples of the acid include edible acids that are used for food or pharmaceuticals, for example, organic acids such as citric acid, malic acid, gluconic acid, tartaric acid, ascorbic acid, succinic acid, lactic acid, acetic acid, fumaric acid, adipic acid, and fumaric acid, and inorganic acids such as phosphoric acid. These acids may be used as a mixture of two or more thereof. In particular, citric acid and/or malic acid which are highly contained in plum vinegar used for salting of red perilla leaves are preferable.

The amount of the acid added in the red perilla leaves to which an acid has been added is not particularly limited within a range that does not impair the effects of the present invention, and is preferably 1 mass% or more and more preferably 10 mass% or more and; is preferably 60 mass% or less and more preferably 40 mass% or less with respect to the dry mass of the red perilla leaves. In addition, the amount is preferably from 1 to 60 mass% and more preferably from 10 to 40 mass%.

[Step (1)]

The step (1) is a step of extracting red perilla leaves to which an acid has been added with water under a condition of a pH of from 4.0 to 8.0.

The pH at the time of extraction is pH of from 4.0 to 8.0 and is, from the viewpoint of collection rate of rosmarinic acid, preferably pH of 4.5 or more and more preferably pH of 5.0 or more; and preferably pH of 7.0 or less and more preferably pH of 6.0 or less. In addition, the pH is preferably pH of from 4.5 to 7.0 and more preferably pH of from 5.0 to 6.0.

Examples of the pH adjuster for adjusting the pH include potassium hydroxide, sodium hydroxide, sodium carbonate, potassium carbonate, and ammonia, and these pH adjusters may be used as a mixture of two or more thereof. In particular, sodium hydroxide is preferable.

The water may be any of distilled water, ion-exchanged water, degassed water, tap water, well water, and so on.

The amount of water to be used in extraction is, as the bath ratio to the red perilla leaves to which an acid has been added (in terms of dry mass), preferably 4 or more and more preferably 6 or more; and preferably 35 or less and more preferably 20 or less. In addition, the amount is preferably from 4 to 35 and more preferably from 6 to 20. Here, in the present specification, the dry mass refers to the mass of the solid residue obtained by lyophilization of red perilla leaves. The bath ratio is a value represented by the ratio of the volume of water to the mass of red perilla leaves to which an acid has been added (in terms of dry mass), [(volume of water)/(mass of red perilla leaves to which an acid has been added (in terms of dry mass))]. Incidentally, the term "volume" in the present invention means the volume at 25°C.

Although the extraction conditions are not particularly limited as long as extraction can be sufficiently performed, for example, the extraction time is preferably 30 minutes or more and more preferably 1 hour or more; and is preferably 10 hours or less and more preferably 2 hours or less. In addition, the extraction time is preferably from 30 minutes to 10 hours and more preferably from 1 to 2 hours.

The extraction can be performed at room temperature or under heating conditions. The extraction temperature under heating conditions is preferably 20°C or more, more preferably 50°C or more, and further preferably 80°C or more; and preferably 100°C or less and more preferably 90°C or less. In addition, the temperature is preferably from 20°C to 100°C, more preferably from 50°C to 100°C, and further preferably from 80°C to 100°C. In general, extraction at low temperature is performed for a long time, and extraction at high temperature is performed for a short time.

Although the extraction means is not particularly limited, for example, ordinary means such as solid-liquid extraction, liquid-liquid extraction, immersion, brewing, percolation, reflux extraction, Soxhlet extraction, ultrasonic extraction, microwave extraction, and stirring can be used.

The extraction step can be performed once or multiple times.

The extract solution obtained by this step may be as it is, may be diluted with an appropriate solvent to be prepared into a diluted solution, or may be prepared into a concentrated solution, dry powder, or paste.

As described below, from the viewpoint of increasing the purity of rosmarinic acid, the extract solution obtained by this step is preferably subjected to a step of concentrating the extract solution and a step of adding ethanol or an ethanol aqueous solution to the concentrated extract solution and then removing a precipitate.

The collection rate of rosmarinic acid from red perilla leaves to which an acid has been added in the extraction step is preferably 80 mass% or more, more preferably 90 mass% or more, and further preferably 95 mass% or more from the viewpoint of production efficiency.

[Step (2)]

The step (2) is a step of concentrating the extract solution obtained in the step (1).

The extract solution can be concentrated by an ordinary means such as atmospheric pressure concentration, reduced pressure concentration, or membrane concentration, and a concentrated extract solution is obtained by using the resultant directly or by a reduction procedure such as dilution with water, as needed.

The solid content concentration of the concentrated extract solution to be subjected to the subsequent step (3) is, from the viewpoint of the collection rate of rosmarinic acid in a purification step and the purity of rosmarinic acid of a purified red perilla leaf extract, preferably 10 mass% or more, more preferably 15 mass% or more, and further preferably 20 mass% or more; and preferably 60 mass% or less, more preferably 50 mass% or less, and further preferably 45 mass% or less. In addition, the solid content concentration is preferably from 10 to 60 mass%, more preferably from 15 to 50 mass%, and further preferably from 20 to 45 mass%. Here, in the present specification, the solid content concentration is a value represented by the ratio of the mass of the solute to the mass of the solution, [solute mass/concentrated extract solution mass]. Incidentally, the mass of the solute refers to the mass of the solid residue obtained by lyophilization of the concentrated extract solution.

The pH of the concentrated extract solution to be subjected to the subsequent step (3) is, from the viewpoint of increasing the purity of rosmarinic acid while leaving rosmarinic acid in the subsequent step (3), pH of 4.0 or more, preferably pH of 4.5 or more, more preferably pH of 5.0 or more, and further preferably pH of 5.5 or more; and preferably pH of 7.5 or less and more preferably pH of 7.0 or less. The pH is preferably pH of from 4.0 to 7.5, more preferably pH of from 4.5 to 7.5, further preferably pH of from 5.0 to 7.0, and further preferably pH of from 5.5 to 7.0. The pH of the concentrated extract solution may be adjusted, as needed, by adjusting the pH of the extract solution before concentration or by adjusting the pH of the concentrated extract solution after concentration. As the pH adjuster for adjusting the pH, for example, one or more of potassium hydroxide, sodium hydroxide, sodium carbonate, potassium carbonate, and ammonia are mentioned. In particular, sodium hydroxide is preferable.

[Step (3)]

Step (3) is a step of adding ethanol or an ethanol aqueous solution to the concentrated extract solution obtained in the step (2) and then removing a precipitate.

The concentration of ethanol in the ethanol aqueous solution is preferably 50 vol% or more, more preferably 70 vol% or more, and further preferably 90 vol% or more, and preferably 99.5 vol% or less, more preferably 98 vol% or less, and further preferably 95 vol% or less. In addition, the concentration is preferably from 50 to 99.5 vol%, more preferably from 70 to 98 vol%, and further preferably from 90 to 95 vol%.

In this step, although the conditions are not particularly limited as long as a precipitate is precipitated by addition of ethanol or an ethanol aqueous solution to the concentrated extract solution and the purity of rosmarinic acid in the solution is increased, from the viewpoint of increasing the purity of rosmarinic acid while leaving rosmarinic acid it is preferable to add ethanol or an ethanol aqueous solution such that the concentration of ethanol with respect to the concentrated extract solution is 40 vol% or more, preferably 55 vol% or more, more preferably 65 vol% or more, and further preferably 70 vol% or more; and 95 vol% or less, preferably 90 vol% or less, more preferably 85 vol% or less, and further preferably 80 vol% or less; and is in a range of from 40 to 95 vol%, preferably from 55 to 90 vol%, more preferably from 65 to 85 vol%, and further preferably from 70 to 80 vol%.

Preferable examples of the steps (2) and (3) are as follows:

- A 50 to 99.5 vol% ethanol aqueous solution is added to a concentrated extract solution having a solid content concentration of from 10 to 60 mass% such that the ethanol concentration is from 40 to 95 vol%;
- A 70 to 98 vol% ethanol aqueous solution is added to a concentrated extract solution having a solid content concentration of from 15 to 50 mass% such that the ethanol concentration is from 55 to 90 vol%;
- A 95 vol% ethanol aqueous solution is added to a concentrated extract solution having a solid content concentration of from 27.5 to 48.7 mass% such that the ethanol concentration is 70 vol%;

- A 95 vol% ethanol aqueous solution is added to a concentrated extract solution having a solid content concentration of from 27.5 to 37.5 mass% and a pH of from 4.5 to 6.7 such that the ethanol concentration is 70 vol%;
- A 95 vol% ethanol aqueous solution is added to a concentrated extract solution having a solid content concentration of from 24.1 to 44.2 mass% such that the ethanol concentration is 65 vol%;
- A 95 vol% ethanol aqueous solution is added to a concentrated extract solution having a solid content concentration of from 24.1 to 38.8 mass% and a pH of from 4.5 to 6.7 such that the ethanol concentration is 65 vol%;
- A 95 vol% ethanol aqueous solution is added to a concentrated extract solution having a solid content concentration of 21.3 to 40.4 mass% such that the ethanol concentration is 60 vol%;
- A 95 vol% ethanol aqueous solution is added to a concentrated extract solution having a solid content concentration of 21.3 to 40.4 mass% and a pH of from 4.5 to 6.7 such that the ethanol concentration is 60 vol%;
- A 95 vol% ethanol aqueous solution is added to a concentrated extract solution having a solid content concentration of 19.2 to 37.3 mass% such that the ethanol concentration is 55 vol%; and
- A 95 vol% ethanol aqueous solution is added to a concentrated extract solution having a solid content concentration of from 27.7 to 32.2 mass% and a pH of from 4.5 to 6.7 such that the ethanol concentration is 55 vol% .

The temperature in this step is preferably 10°C or more and more preferably 20°C or more; and preferably 40°C or less and more preferably 30°C or less; and is preferably from 10°C to 40°C and more preferably from 20°C to 30°C.

The method for removing the precipitated precipitate is not particularly limited, and the method can be performed by centrifugation, decantation, or filtration.

The extract solution containing rosmarinic acid prepared by removing the precipitate precipitated in this step may be as it is or may be prepared to a concentrated solution, dry powder, or paste.

Thus, a purified red perilla leaf extract having a high purity of rosmarinic acid is obtained with a high yield.

In the purified red perilla leaf extract of the present invention through the steps (1) to (3), the purity of rosmarinic acid can be made 8 mass% or more, preferably 11 mass% or more, and more preferably 14 mass% or more. The purification collection rate of rosmarinic acid from the concentrated extract solution can be made a high yield, preferably 80 mass% or more, more preferably 90 mass% or more, and further preferably 95 mass% or more.

The method for producing a red perilla leaf extract of the present invention is, from the viewpoint of the collection rate of rosmarinic acid, preferably a method for producing a red perilla leaf extract including the following step (1):

(1) a step of extracting red perilla leaves to which an acid has been added with water under a condition of a pH of from 5.0 to 8.0 at an extraction temperature of from 20°C to 90°C and a bath ratio of water of from 4 to 35 (L/kg).

The method for producing a purified red perilla leaf extract of the present invention is, from the viewpoint of the collection rate of rosmarinic acid and the viewpoint of increasing the purity of rosmarinic acid while leaving rosmarinic acid, preferably a method for producing a purified red perilla leaf extract including the following steps (1) to (3):

(1) a step of extracting red perilla leaves to which an acid has been added with water under a condition of a pH of from 4.0 to 8.0;
(2) a step of concentrating the extract solution obtained in the step (1), wherein, as needed, the pH of the extract solution obtained in the step (1) is adjusted to a pH of from 4.5 to 7.0 before concentration or the pH of the concentrated extract solution is adjusted to a pH of from 4.5 to 7.0 after concentration; and
(3) a step of adding ethanol or an ethanol aqueous solution to the concentrated extract solution having a pH of from 4.5 to 7.0 obtained in the step (2) such that the ethanol concentration is from 40 to 95 vol% and then removing a precipitate.

The method for producing a purified red perilla leaf extract of the present invention is, from the viewpoint of the collection rate of rosmarinic acid and the viewpoint of increasing the purity of rosmarinic acid while leaving rosmarinic acid, preferably a method for producing a purified red perilla leaf extract including the following steps (1) to (3):

(1) a step of extracting red perilla leaves to which an acid has been added with water under a condition of a pH of from 4.0 to 8.0;

(2) a step of concentrating the extract solution obtained in the step (1) to obtain a concentrated extract solution having a solid content concentration of from 15 to 50 mass%, wherein, as needed, the pH of the extract solution obtained in the step (1) is adjusted to a pH of from 4.5 to 7.0 before concentration or the pH of the concentrated extract solution is adjusted to a pH of from 4.5 to 7.0 after concentration; and

(3) a step of adding ethanol or an ethanol aqueous solution to the concentrated extract solution having a solid content concentration of from 15 to 50 mass% and a pH of from 4.5 to 7.0 obtained in the step (2) such that the ethanol concentration is from 40 to 95 vol% and then removing a precipitate.

The method for producing a purified red perilla leaf extract of the present invention is, from the viewpoint of the collection rate of rosmarinic acid and the viewpoint of increasing the purity of rosmarinic acid while leaving rosmarinic acid, preferably a method for producing a purified red perilla leaf extract including the following steps (1) to (3):

(1) a step of extracting red perilla leaves to which an acid has been added with water under a condition of a pH of from 4.0 to 8.0 at an extraction temperature of from 20°C to 90°C and a bath ratio of water of from 4 to 35 (L/kg);

(2) a step of concentrating the extract solution obtained in the step (1) to obtain a concentrated extract solution having a solid content concentration of from 15 to 50 mass%, wherein, as needed, the pH of the extract solution obtained in the step (1) is adjusted to a pH of from 4.5 to 7.0 before concentration or the pH of the concentrated extract solution is adjusted to a pH of from 4.5 to 7.0 after concentration; and

(3) a step of adding ethanol or an ethanol aqueous solution to the concentrated extract solution having a solid content concentration of from 15 to 50 mass% and a pH of from 4.5 to 7.0 obtained in the step (2) such that the ethanol concentration is from 40 to 95 vol% and then removing a precipitate.

The method for producing a purified red perilla leaf extract of the present invention is, from the viewpoint of the collection rate of rosmarinic acid and the viewpoint of increasing the purity of rosmarinic acid while leaving rosmarinic acid, preferably a method for producing a purified red perilla leaf extract including the following steps (1) to (3):

(1) a step of extracting red perilla leaves to which an acid has been added with water under a condition of a pH of from 4.0 to 8.0 at an extraction temperature of from 50°C to 100°C and a bath ratio of water of from 6 to 20 (L/kg);

(2) a step of concentrating the extract solution obtained in the step (1) to obtain a concentrated extract solution having a solid content concentration of from 15 to 50 mass%, wherein, as needed, the pH of the extract solution obtained in the step (1) is adjusted to a pH of from 4.5 to 7.5 before concentration or the pH of the concentrated extract solution is adjusted to a pH of from 4.5 to 7.5 after concentration; and

(3) a step of adding ethanol or an ethanol aqueous solution to the concentrated extract solution having a solid content concentration of from 15 to 50 mass% and a pH of from 4.5 to 7.5 obtained in the step (2) such that the ethanol concentration is from 55 to 90 vol% and then removing a precipitate.

The method for producing a purified red perilla leaf extract of the present invention is, from the viewpoint of the collection rate of rosmarinic acid and the viewpoint of increasing the purity of rosmarinic acid while leaving rosmarinic acid, preferably a method for producing a purified red perilla leaf extract including the following steps (1) to (3):

(1) a step of extracting red perilla leaves to which an acid has been added with water under a condition of a pH of from 4.0 to 8.0 at an extraction temperature of from 80°C to 100°C and a bath ratio of water of from 6 to 20 (L/kg);

(2) a step of concentrating the extract solution obtained in the step (1) to obtain a concentrated extract solution having a solid content concentration of from 15 to 50 mass%, wherein, as needed, the pH of the extract solution obtained in the step (1) is adjusted to a pH of from 5.0 to 7.0 before concentration or the pH of the concentrated extract solution is adjusted to a pH of from 5.0 to 7.0 after concentration; and

(3) a step of adding ethanol or an ethanol aqueous solution to the concentrated extract solution having a solid content concentration of from 15 to 50 mass% and a pH of from 5.0 to 7.0 obtained in the step (2) such that the ethanol concentration is from 65 to 85 vol% and then removing a precipitate.

Regarding the above-described embodiments, the present invention further discloses the following aspects:
<1> A method for producing a red perilla leaf extract comprising the following step (1):

(1) a step of extracting red perilla leaves to which an acid has been added with water under a condition of a pH of from 4.0 to 8.0;

<2> A method for producing a purified red perilla leaf extract comprising the following steps (1) to (3):

(1) a step of extracting red perilla leaves to which an acid has been added with water under a condition of a pH of from 4.0 to 8.0;
(2) a step of concentrating the extract solution obtained in the step (1); and
(3) a step of adding ethanol or an ethanol aqueous solution to the concentrated extract solution obtained in the step (2) and then removing a precipitate;

<3> The producing method according to <1> or <2>, wherein the amount of the acid added in the red perilla leaves to which an acid has been added is preferably 1 mass% or more, more preferably 10 mass% or more; and preferably 60 mass% or less and more preferably 40 mass% or less; and is preferably from 1 to 60 mass% and more preferably from 10 to 40 mass%;
<4> The producing method according to any one of <1> to <3>, wherein the pH at the time of extraction in the step (1) is preferably pH of 4.5 or more and more preferably pH of 5.0 or more; and preferably pH of 7.0 or less and more preferably pH of 6.0 or less; and is preferably pH of from 4.5 to 7.0 and more preferably pH of from 5.0 to 6.0;
<5> The producing method according to any one of <1> to <4>, wherein the bath ratio of water in the step (1) is preferably 4 or more and more preferably 6 or more; and preferably 35 or less and more preferably 20 or less; and is preferably from 4 to 35 and more preferably from 6 to 20;
<6> The producing method according to any one of <1> to <5>, wherein the extraction time in the step (1) is preferably 30 minutes or more and more preferably 1 hour or more; and preferably 10 hours or less and more preferably 2 hours or less; and is preferably from 30 minutes to 10 hours and more preferably from 1 to 2 hours;
<7> The producing method according to any one of <1> to <6>, wherein the extraction temperature in the step (1) is preferably 20°C or more, more preferably 50°C or more and more preferably 80°C or more; and preferably 100°C or less; and is preferably from 20°C to 100°C, more preferably from 50°C to 100°C, and further preferably from 80°C to 100°C;
<8> The producing method according to any one of <2> to <7>, wherein, in the step (2), further, the pH of the extract solution obtained in the step (1) is adjusted before concentration to preferably pH of 4.0 or more, more preferably pH of 4.5 or more, further preferably pH of 5.0 or more, and further preferably pH of 5.5 or more; and preferably pH of 7.5 or less and more preferably pH of 7.0 or less; and to preferably pH of from 4.0 to 7.5, more preferably pH of from 4.5 to 7.5, further preferably pH of from 5.0 to 7.0, and further preferably pH of from 5.5 to 7.0, or the pH of the concentrated extract solution is adjusted after concentration to preferably pH of 4.0 or more, more preferably pH of 4.5 or more, further preferably pH of 5.0 or more, and further preferably pH of 5.5 or more; and preferably pH of 7.5 or less and more preferably pH of from 7.0 or less; and to preferably pH of from 4.0 to 7.5, more preferably pH of from 4.5 to 7.5, further preferably pH of from 5.0 to 7.0, and further preferably pH of from 5.5 to 7.0, as needed;
<9> The producing method according to any one of <2> to <8>, wherein, in the step (3), the pH of the concentrated extract solution is preferably pH of 4.0 or more, more preferably pH of 4.5 or more, further preferably pH of 5.0 or more, and further preferably pH of 5.5 or more; and preferably pH of 7.5 or less and more preferably pH of 7.0 or less; and is preferably pH of from 4.0 to 7.5, more preferably pH of from 4.5 to 7.5, further preferably pH of from 5.0 to 7.0, and further preferably pH of from 5.5 to 7.0;
<10> The producing method according to any one of <2> to <9>, wherein, in the step (3), the solid content concentration in the concentrated extract solution is preferably 10 mass% or more, more preferably 15 mass% or more, and further preferably 20 mass% or more; and preferably 60 mass% or less, more preferably 50 mass% or less, and further preferably 45 mass% or less; and is preferably from 10 to 60 mass%, more preferably from 15 to 50 mass%, and further preferably from 20 to 45 mass%;
<11> The producing method according to any one of <2> to <10>, wherein, in the step (3), the ethanol concentration in the ethanol aqueous solution is preferably 50 vol% or more, more preferably 70 vol% or more, and further preferably 90 vol% or more; and preferably 99.5 vol% or less, more preferably 98 vol% or less, and further preferably 95 vol% or less; and is preferably from 50 to 99.5 vol%, more preferably from 70 to 98 vol%, and further preferably from 90 to 95 vol%;
<12> The producing method according to any one of <2> to <11>, wherein, in the step (3), ethanol or an ethanol aqueous solution is added to the concentrated extract solution such that the ethanol concentration is preferably 40 vol% or more, more preferably 55 vol% or more, further preferably 65 vol% or more, and further preferably 70 vol% or more; and preferably 95 vol% or less, more preferably 90 vol% or less, more preferably 85 vol% or less, and further preferably 80 vol% or less; and is preferably in a range of from 40 to 95 vol%, more preferably from 55 to 90 vol%, further preferably from 65 to 85 vol%, and further preferably from 70 to 80 vol%;
<13> The producing method according to any one of <2> to <12>, wherein the addition of ethanol or an ethanol aqueous solution to the concentrated extract solution in the step (3) is any of the followings:

- A 50 to 99.5 vol% ethanol aqueous solution is added to a concentrated extract solution having a solid content concentration of from 10 to 60 mass% such that the ethanol concentration is from 40 to 95 vol%;
- A 70 to 98 vol% ethanol aqueous solution is added to a concentrated extract solution having a solid content concentration of from 15 to 50 mass% such that the ethanol concentration is from 55 to 90 vol%;
- A 95 vol% ethanol aqueous solution is added to a concentrated extract solution having a solid content concentration of 27.5 to 48.7 mass% such that the ethanol concentration is 70 vol%;
- A 95 vol% ethanol aqueous solution is added to a concentrated extract solution having a solid content concentration of from 27.5 to 37.5 mass% and a pH of from 4.5 to 6.7 such that the ethanol concentration is 70 vol%;
- A 95 vol% ethanol aqueous solution is added to a concentrated extract solution having a solid content concentration of from 24.1 to 44.2 mass% such that the ethanol concentration is 65 vol%;
- A 95 vol% ethanol aqueous solution is added to a concentrated extract solution having a solid content concentration of from 24.1 to 38.8 mass% and a pH of from 4.5 to 6.7 such that the ethanol concentration is 65 vol%;
- A 95 vol% ethanol aqueous solution is added to a concentrated extract solution having a solid content concentration of from 21.3 to 40.4 mass% such that the ethanol concentration is 60 vol%;
- A 95 vol% ethanol aqueous solution is added to a concentrated extract solution having a solid content concentration of from 21.3 to 40.4 mass% and a pH of from 4.5 to 6.7 such that the ethanol concentration is 60 vol%;
- A 95 vol% ethanol aqueous solution is added to a concentrated extract solution having a solid content concentration of from 19.2 to 37.3 mass% such that the ethanol concentration is 55 vol%; and
- A 95 vol% ethanol aqueous solution is added to a concentrated extract solution having a solid content concentration of from 27.7 to 32.2 mass% and a pH of from 4.5 to 6.7 such that the ethanol concentration is 55 vol%;

<14> The producing method according to any one of <2> to <13>, wherein the temperature in the step (3) is preferably 10°C or more and more preferably 20°C or more; and preferably 40°C or less and more preferably 30°C or less; and is preferably from 10°C to 40°C and more preferably from 20°C to 30°C; and

<15> The producing method according to any one of <2> to <14>, wherein the purity of rosmarinic acid in the purified red perilla leaf extract is preferably 8 mass% or more, more preferably 11 mass% or more, and further preferably 14 mass% or more.

Examples

(i) Rosmarinic acid (RA) analysis

The amounts of RA in samples were measured by HPLC (high-performance liquid chromatography). First, a sample was weighed in a tube, and an aqueous solution having a solid content concentration of 1 vol% was prepared. This aqueous solution was appropriately diluted with a 50 vol% methanol aqueous solution, and filtration was then performed with 0.45 $\mu$m filter (PES, Agilent Technologies, Inc.), followed by analysis.

The standard for concentration calibration used was reagent RA (produced by Carbosynth Limited). As the analysis column, Capcell Core C18 (2.1 mm $\times$ 100 mm, 2.7 $\mu$m, produced by Osaka Soda Co., Ltd.) was used, and analysis was performed by setting the flow rate to 0.7 mL/min, the column temperature to 40°C, the sample injection amount to 10 $\mu$L, and the UV detection wavelength to 320 nm. As the eluent, a 0.1 vol% formic acid aqueous solution as solution A and acetonitrile as solution B were used, and the analysis was implemented by a gradient system of the two solutions. The gradient conditions were as follows:

| Time (min) (vol%) | solution A (vol%) | solution B |
| --- | --- | --- |
| 0-0.3 | 94 | 6 |
| 0.3-1.2 | 82 | 18 |
| 1.2-2.2 | 82 | 18 |
| 2.2-2.7 | 75 | 25 |
| 2.7-4.5 | 75 | 25 |
| 4.5-5.5 | 3 | 97 |
| 5.5-8.0 | 94 | 6 |

(ii) Measurement of pH

pH was measured at room temperature using LAQUA pH/ION METER (produced by HORIBA, Ltd.) and GRT composite electrode (produced by HORIBA, Ltd.).

(iii) RA collection rate

The RA collection rate of a red perilla leaf extract was determined using the mass of RA in red perilla leaves to which an acid has been added as 100 mass% by the following expression:

RA collection rate [mass%] = (mass of RA in red
perilla leaf extract)/(mass of RA in red perilla leaves
to which an acid has been added) × 100,

(mass of RA in red perilla leaves to which an acid
has been added) = (RA content rate in red perilla leaves
to which an acid has been added) × (mass of charged red
perilla leaves to which an acid has been added).

Incidentally, the RA content rate [mass%] in red perilla leaves to which an acid has been added was calculated by the following method.

5 g of red perilla leaves to which an acid has been added and 50 mL of methanol (bath ratio: 10) were put in a 100-mL conical flask, followed by leaving to stand at room temperature for extraction for 8 days. Subsequently, the extract solution was filtered through a GFP filter (Kiriyama glass Co., Ltd.), and the filtration residue was further washed with 50 mL of methanol (bath ratio: 10). The extract solution and the washing liquid were combined, followed by diluting in a measuring cylinder to 100 mL. The RA content was measured by HPLC by the same method as above.

RA content rate [mass%] in red perilla leaves to
which an acid has been added = (mass of RA in methanol
extract)/(mass of charged red perilla leaves to which an
acid has been added) × 100

(iv) Purification collection rate of RA

The purification collection rate of RA in a purified red perilla leaf extract was determined by the following expression:

Purification collection rate [mass%] of RA = (mass
of RA in purified red perilla leaf extract)/(mass of RA
in red perilla leaf extract before addition of ethanol
aqueous solution) × 100.

(v) RA purity

The RA purity was determined using the mass of solid content in a sample as 100 mass% by the following expression:

$$\text{RA purity [mass\%]} = \text{(mass of RA in sample)/(mass of solid content in sample)} \times 100$$

Example 1

The red perilla leaves to which an acid has been added used as an extraction raw material were dry red perilla leaf powder to which citric acid has been added (red perilla powder KU, produced by Kodama Foods Co., Ltd.). The RA content rate in the extraction raw material was 2.50 mass%.

40 mL of water was added to 2 g of the extraction raw material (bath ratio: 20), and an 8 N NaOH aqueous solution was dropwise added thereto to adjust the pH to 4.0. Subsequently, extraction was performed with stirring under a heating condition of 80°C for 1 hour, and the extract solution was collected by filtration. Furthermore, 30 mL of room temperature water (bath ratio: 15) was added to the filtration residue for filtration to collect RA remaining in the residue. The collected extract solutions were combined and were then lyophilized to obtain a dry powder of the red perilla leaf extract.

Th RA content in the obtained extract was measured to determine the RA collection rate.

Examples 2 to 7

Dry powders of red perilla leaf extracts were obtained from the extraction raw material by performing extraction as in Example 1 under conditions of pH and temperature shown in Table 1.

The RA contents in the obtained extracts were measured to determine the RA collection rates.

Example 8

200 mL of water was added to 20 g of an extraction raw material similar to that of Example 1 (bath ratio: 10), and an 8 N NaOH aqueous solution was dropwise added thereto to adjust the pH to 6.0. Subsequently, extraction was performed with stirring under a heating condition of 90°C for 1 hour, and the extract solution was collected by filtration. Furthermore, 200 mL of room temperature water (bath ratio: 10) was added to the filtration residue for filtration to collect RA remaining in the residue. The collected extract solutions were combined and were then lyophilized to obtain a dry powder of the red perilla leaf extract. The total bath ratio of the used extraction solvent was 20.

The RA content in the obtained extract was measured to determine the RA collection rate.

Example 9

160 mL of water was added to 20 g of an extraction raw material similar to that of Example 1 (bath ratio: 8), and an 8 N NaOH aqueous solution was dropwise added thereto to adjust the pH to 6.0. Subsequently, extraction was performed with stirring under a heating condition of 90°C for 1 hour, and the extract solution was collected by filtration. Furthermore, 160 mL of room temperature water (bath ratio: 8) was added to the filtration residue for filtration to collect RA remaining in the residue. The collected extract solutions were combined and were then lyophilized to obtain a dry powder of the red perilla leaf extract. The total bath ratio of the used extraction solvent was 16.

The RA content in the obtained extract was measured to determine the RA collection rate.

Example 10

120 mL of water was added to 20 g of an extraction raw material similar to that of Example 1 (bath ratio: 6), and an 8 N NaOH aqueous solution was dropwise added thereto to adjust the pH to 6.0. Subsequently, extraction was performed with stirring under a heating condition of 90°C for 1 hour, and the extract solution was collected by filtration. Furthermore, 120 mL of room temperature water (bath ratio: 6) was added to the filtration residue for filtration to collect RA remaining in the residue. The collected extract solutions were combined and were then lyophilized to obtain a dry powder of the red perilla leaf extract. The total bath ratio of the used extraction solvent was 12.

The RA content in the obtained extract was measured to determine the RA collection rate.

Comparative Example 1

40 mL of water was added to 2 g of an extraction raw material similar to that of Example 1 (bath ratio: 20), and subsequently, extraction was performed with stirring under a heating condition of 80°C for 1 hour, and the extract solution was collected by filtration. The pH at the time of extraction was 2.9. Furthermore, 30 mL of room temperature water (bath ratio: 15) was added to the filtration residue for filtration to collect RA remaining in the residue. The collected extract solutions were combined and were then lyophilized to obtain a dry powder of the red perilla leaf extract.

The RA content in the obtained extract was measured to determine the RA collection rate.

The treatment conditions and the results of analysis in Examples and Comparative Example are shown in Table 1.

[Table 1]

| | pH at the time of extraction | Temperature at the time of extraction [°C] | Total bath ratio of water | RA collection rate [W/W] |
|---|---|---|---|---|
| Example 1 | 4.0 | 80 | 35 | 68% |
| Example 2 | 5.0 | | | 91% |
| Example 3 | 6.0 | | | 88% |
| Example 4 | 7.0 | | | 81% |
| Example 5 | 8.0 | | | 78% |
| Example 6 | 5.0 | 20 | 35 | 72% |
| Example 7 | | 50 | | 82% |
| Example 8 | 6.0 | 90 | 20 | 88% |
| Example 9 | | | 16 | 75% |
| Example 10 | | | 12 | 86% |
| Comparative Example 1 | 2.9 | 80 | 35 | 56% |

It was confirmed from Table 1 that at an extraction temperature of from 20°C to 90°C, when the pH at the time of extraction is 4.0 to 8.0, the RA collection rate is improved compared to Comparative Example 1. In particular, a pH range of 5.0 to 7.0 was preferable. Furthermore, the extraction temperature of 50°C or more was preferable.

Example 11

20 g of an extraction raw material similar to that of Example 1 and 200 mL of water (bath ratio: 10) were put in a centrifuge tube, and an 8 N NaOH aqueous solution was dropwise added thereto to adjust the pH to 6.0. Subsequently, extraction was performed with stirring under a heating condition of 50°C for 1 hour. Subsequently, the centrifuge tube was centrifuged at 3,000 r/min at 20°C for 5 minutes, and the supernatant was collected. Furthermore, 200 mL of room temperature water (bath ratio: 10) was added to the residue, and extraction with stirring and centrifugation were performed under the same conditions, and the supernatant was collected. This operation was repeated once again, and the supernatants for a total of three times were combined. This extract solution was lyophilized to obtain a dry powder of the red perilla leaf extract.

The RA content in the obtained extract was measured, and as a result, the RA collection rate was 97 mass%. The RA purity was 4.02 mass%.

Subsequently, 200 mg of the dry powder of the red perilla leaf extract was weighed in a tube, and 500 μL of water was added thereto, followed by stirring to prepare a concentrated extract solution having a solid content concentration of 28.6 mass%.

To this concentrated extract solution, 1.4 mL of a 95 vol% ethanol aqueous solution was added at room temperature such that the final ethanol concentration was 70 vol% to form an insoluble precipitate. Subsequently, centrifugation was performed at 3,000 r/min at 20°C for 10 minutes, and the supernatant was collected. Furthermore, half the amount of the ethanol aqueous solution at the time of precipitate formation was added to the remaining precipitate, and the precipitate was dispersed with a spatula.

Incidentally, the ethanol concentration in the ethanol aqueous solution at this time was adjusted to the final ethanol concentration at the time of precipitate formation. After centrifugation under the same conditions, the supernatant was collected and was combined with the supernatant collected earlier. The combined supernatants were concentrated and then dried to obtain a purified red perilla leaf extract.

The RA content in the obtained purified red perilla leaf extract was measured, and the RA purification collection rate and the RA purity were determined.

Examples 12 and 13

Purified red perilla leaf extracts were prepared from an extraction raw material by performing the same treatment as in Example 11 except that water and the ethanol aqueous solution were added in amounts such that the solid content concentrations of the concentrated extract solutions were the values shown in Table 2.
The RA content in each of the obtained purified red perilla leaf extracts was measured, and the RA purification collection rate and the RA purity were determined.

Example 14

20 mL of room temperature water was added to 4 g of an extraction raw material similar to that of Example 1 (bath ratio: 5), and an 8 N NaOH aqueous solution was dropwise added thereto to adjust the pH to 4.5. Subsequently, extraction was performed with stirring under a heating condition of 50°C for 1 hour. Subsequently, the centrifuge tube was centrifuged at 3,000 r/min at 20°C for 5 minutes, and the supernatant was collected. Furthermore, 20 mL of room temperature water (bath ratio: 5) was added to the residue, and extraction with stirring and centrifugation were performed under the same conditions, and the supernatant was collected. This operation was repeated once again, and the supernatants for a total of three times were combined. This extract solution was lyophilized to obtain a dry powder of the red perilla leaf extract.
The RA content in the obtained extract was measured, and as a result, the RA collection rate was 83 mass%. The RA purity was 4.04 mass%.
Subsequently, 200 mg of the dry powder of the red perilla leaf extract was weighed in a tube, and 500 $\mu$L of water was added thereto, followed by stirring to prepare a concentrated extract solution having a solid content concentration of 28.6 mass%.
This concentrated extract solution was subjected to the same treatment as in Example 11 to obtain a purified red perilla leaf extract from the extraction raw material.
The RA content in the obtained purified red perilla leaf extract was measured, and the RA purification collection rate and the RA purity were determined.

Examples 15 and 16

Purified red perilla leaf extracts were prepared from an extraction raw material by performing the same treatment as in Example 14 except that the solid content concentrations of the concentrated extract solutions were adjusted to the values shown in Table 2.
The RA content in each of the obtained purified red perilla leaf extracts was measured, and the RA purification collection rate and the RA purity were determined.

Examples 17 to 20

2 L of water was added to 200 g of an extraction raw material similar to that of Example 1 (bath ratio: 10), and a 6 N NaOH aqueous solution was dropwise added thereto to adjust the pH to 4.5. Subsequently, extraction was performed with stirring under a heating condition of 90°C for 1 hour. The extract solution was then filtered through a sieve (aperture: 106 $\mu$m, wire diameter: 71 $\mu$m), and the obtained filtrate was filtered through a filter paper (No. 2 size, produced by Agilent Technologies, Inc.). The obtained extract solution was concentrated under reduced pressure at 40°C to obtain a red perilla leaf extract as the concentrated extract solution.
The RA content and solid content in the obtained extract were measured, and as the results, the RA purity was 4.33 mass%, and the solid content concentration was 28.5 mass%.
Subsequently, 800 mg of the concentrated extract solution was weighed in a tube, and an 8 N NaOH aqueous solution was added thereto to adjust the pH of the concentrated extract solution to the value shown in Table 2. A 95 vol% ethanol aqueous solution was added to this concentrated extract solution at room temperature such that the final ethanol concentration was 70 vol%. Subsequent treatment was performed as in Example 11 to prepare a purified red perilla leaf extract from the extraction raw material.
The RA content in each of the obtained purified red perilla leaf extracts was measured, and the RA purification collection rate and the RA purity were determined.

Example 21

150 mL of water was added to 30 g of an extraction raw material similar to that of Example 1 (bath ratio: 5), and an 8 N NaOH aqueous solution was dropwise added thereto to adjust the pH to 4.5. Subsequently, extraction was performed with stirring under a heating condition of 90°C for 1 hour. Subsequently, the centrifuge tube was centrifuged at 3,000 r/min at 20°C for 5 minutes, and the supernatant was collected. Furthermore, 150 mL of water (bath ratio: 5) was added to the residue, and extraction with stirring and centrifugation were performed under the same conditions, and the supernatant was collected. This operation was repeated once again, and the supernatants for a total of three times were combined. An 8 N NaOH aqueous solution was dropwise added to this extract solution to adjust the pH to 6.0, followed by lyophilization to obtain a dry powder of the red perilla leaf extract.

The RA content in the obtained extract was measured, and as a result, the RA collection rate was 94 mass%. The RA purity was 4.12 mass%.

Subsequently, 200 mg of the dry powder of the red perilla leaf extract was weighed in a tube, and 526 μL of water was added thereto, followed by stirring to prepare a concentrated extract solution having a solid content concentration of 27.5 mass%.

This concentrated extract solution was subjected to the same treatment as in Example 11 to obtain a purified red perilla leaf extract from the extraction raw material.

The RA content in the obtained purified red perilla leaf extract was measured, and the RA purification collection rate and the RA purity were determined.

Examples 22 to 25

Purified red perilla leaf extracts were prepared from an extraction raw material by performing the same treatment as in Example 21 except that water was added in amounts such that the solid content concentrations of the concentrated extract solutions were adjusted to the values shown in Table 3.

The RA content in each of the obtained purified red perilla leaf extracts was measured, and the RA purification collection rate and the RA purity were determined.

Examples 26 to 30

Purified red perilla leaf extracts were prepared from an extraction raw material by performing the same treatment as in Example 21 except that water was added in amounts such that the solid content concentrations of the concentrated extract solutions were the values shown in Table 3 and that a 95 vol% ethanol aqueous solution was added such that the final ethanol concentration was 65 vol%.

The RA content in each of the obtained purified red perilla leaf extracts was measured, and the RA purification collection rate and the RA purity were determined.

Examples 31 to 35

Purified red perilla leaf extracts were prepared from an extraction raw material by performing the same treatment as in Example 21 except that water was added in amounts such that the solid content concentrations of the concentrated extract solutions were the values shown in Table 3 and that a 95 vol% ethanol aqueous solution was added such that the final ethanol concentration was 60 vol%.

The RA content in each of the obtained purified red perilla leaf extracts was measured, and the RA purification collection rate and the RA purity were determined.

Examples 36 to 40

Purified red perilla leaf extracts were prepared from an extraction raw material by performing the same treatment as in Example 21 except that water was added in amounts such that the solid content concentrations of the concentrated extract solutions were the values shown in Table 3 and that a 95 vol% ethanol aqueous solution was added such that the final ethanol concentration was 55 vol%.

The RA content in each of the obtained purified red perilla leaf extracts was measured, and the RA purification collection rate and the RA purity were determined.

The treatment conditions and the results of analysis in Examples are shown in Tables 2 and 3.

[Table 2]

| | Solid content concentration of concentrated extract solution [W/W] | Concentrated extract solution pH | Final ethanol concentration [V/V] | RA purification collection rate [W/W] | RA purity [W/W] |
|---|---|---|---|---|---|
| Example 11 | 28.6% | 6.0 | 70% | > 98% | 16.0% |
| Example 12 | 37.5% | | | > 98% | 19.1% |
| Example 13 | 44.4% | | | 98% | 19.4% |
| Example 14 | 28.6% | 4.5 | 70% | 94% | 9.2% |
| Example 15 | 37.5% | | | > 98% | 12.2% |
| Example 16 | 44.4% | | | 83% | 12.5% |
| Example 17 | 28.5% | 4.9 | 70% | 98% | 11.5% |
| Example 18 | | 5.5 | | > 98% | 15.9% |
| Example 19 | | 6.1 | | 97% | 17.6% |
| Example 20 | | 6.7 | | 91% | 17.1% |

[Table 3]

| | Solid content concentration of concentrated extract solution [W/W] | Concentrated extract solution pH | Final ethanol concentration [V/V] | RA purification collection rate [W/W] | RA purity [W/W] |
|---|---|---|---|---|---|
| Example 21 | 27.5% | 6.0 | 70% | > 98% | 16.6% |
| Example 22 | 33.6% | | | 95% | 15.5% |
| Example 23 | 38.0% | | | 88% | 14.4% |
| Example 24 | 43.2% | | | 90% | 17.1% |
| Example 25 | 48.7% | | | 79% | 18.0% |
| Example 26 | 24.1% | | 65% | > 98% | 14.3% |
| Example 27 | 29.7% | | | > 98% | 14.6% |
| Example 28 | 33.8% | | | 96% | 15.9% |
| Example 29 | 38.8% | | | 97% | 13.9% |
| Example 30 | 44.2% | | | 88% | 18.0% |
| Example 31 | 21.3% | | 60% | > 98% | 9.4% |
| Example 32 | 26.6% | | | > 98% | 11.2% |
| Example 33 | 30.4% | | | > 98% | 13.7% |
| Example 34 | 35.2% | | | 96% | 14.1% |
| Example 35 | 40.4% | | | 94% | 16.3% |
| Example 36 | 19.2% | | 55% | > 98% | 6.3% |
| Example 37 | 24.1% | | | > 98% | 7.8% |
| Example 38 | 27.7% | | | 96% | 9.3% |
| Example 39 | 32.2% | | | 97% | 11.2% |
| Example 40 | 37.3% | | | 87% | 12.1% |

It was confirmed from Tables 2 and 3 that RA can be highly purified while preventing a loss in the purification process and efficiently collecting RA by concentrating an extract solution and then performing ethanol precipitation. In particular, the solid content concentration and pH of a concentrated extract solution to be subjected to ethanol precipitation and the final ethanol concentration were preferably in the following ranges:

- Solid content concentration: 27.5 mass% to 37.5 mass%, pH: 4.5 to 6.7, and final ethanol concentration: 70 vol%;
- Solid content concentration: 24.1 mass% to 38.8 mass%, pH: 4.5 to 6.7, and final ethanol concentration: 65 vol%;
- Solid content concentration: 21.3 mass% to 40.4 mass%, pH: 4.5 to 6.7, and final ethanol concentration: 60 vol%; and
- Solid content concentration: 27.7 mass% to 32.2 mass%, pH: 4.5 to 6.7, and final ethanol concentration: 55 vol%.

**Claims**

1. A method for producing a red perilla leaf extract, comprising the following step (1):

   (1) a step of extracting red perilla leaves to which an acid has been added with water under a condition of a pH of from 4.0 to 8.0.

2. A method for producing a purified red perilla leaf extract, comprising following steps (1) to (3):

   (1) a step of extracting red perilla leaves to which an acid has been added with water under a condition of a pH of from 4.0 to 8.0;
   (2) a step of concentrating the extract solution obtained in the step (1); and
   (3) a step of adding ethanol or an ethanol aqueous solution to the concentrated extract solution obtained in the step (2) and then removing a precipitate.

3. The producing method according to claim 1 or 2, wherein an extraction temperature in the step (1) is 50°C or more.

4. The producing method according to any one of claims 1 to 3, wherein, in the step (1), a bath ratio of water is from 4 to 35 (L/kg).

5. The producing method according to any one of claims 2 to 4, wherein, in the step (3), ethanol or an ethanol aqueous solution is added to the concentrated extract solution such that an ethanol concentration is from 40 to 95 vol%.

6. The producing method according to any one of claims 2 to 5, wherein, in the step (2), the pH of the extract solution obtained in the step (1) is further optionally adjusted to a pH of from 4.0 to 7.5 before concentration or the pH of the concentrated extract solution is further optionally adjusted to a pH of from 4.0 to 7.5 after concentration.

7. The producing method according to any one of claims 2 to 6, wherein, in the step (3), the concentrated extract solution has a pH of from 4.0 to 7.5.

**INTERNATIONAL SEARCH REPORT**

| International application No. |
|---|
| PCT/JP2019/046059 |

| A. CLASSIFICATION OF SUBJECT MATTER |
|---|
| Int. Cl. A61K31/216(2006.01)i, A61P29/00(2006.01)i, A61P37/08(2006.01)i, A61K36/535(2006.01)i, A23L19/00(2016.01)i, A23L33/105(2016.01)i<br>FI: A61K36/535, A61P37/08, A61P29/00, A61K31/216, A23L19/00 A, A23L33/105<br>According to International Patent Classification (IPC) or to both national classification and IPC |

| B. FIELDS SEARCHED |
|---|
| Minimum documentation searched (classification system followed by classification symbols)<br>Int. Cl. A61K31/216, A61P29/00, A61P37/08, A61K36/535, A23L19/00, A23L33/105 |
| Documentation searched other than minimum documentation to the extent that such documents are included in the fields searched<br>Published examined utility model applications of Japan    1922-1996<br>Published unexamined utility model applications of Japan    1971-2020<br>Registered utility model specifications of Japan    1996-2020<br>Published registered utility model applications of Japan    1994-2020 |
| Electronic data base consulted during the international search (name of data base and, where practicable, search terms used)<br>JSTPlus/JMEDPlus/JST7580 (JDreamIII);<br>CAplus/REGISTRY/MEDLINE/EMBASE/BIOSIS/WPIDS (STN) |

| C. DOCUMENTS CONSIDERED TO BE RELEVANT | | |
|---|---|---|
| Category* | Citation of document, with indication, where appropriate, of the relevant passages | Relevant to claim No. |
| Y | WO 2002/062365 A1 (MEIJI SEIKA KAISHA, LTD.) 15 August 2002, pages 7, 8 | 1-7 |
| Y | こだま食品株式会 赤しそパウダー(有塩) 商品情報 [online] [retrieved: 27 January 2020], Internet: <URL:https://www.kodama-foods.co.jp/gyoumu/leafyvege/lv_akashiso/aka_3.html> entire text, (KODAMA FOODS CO., LTD. Red shiso powder (Salted). Product Information.) | 1-7 |

☒ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>27.01.2020 | Date of mailing of the international search report<br>10.02.2020 |
|---|---|
| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2019/046059 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | Amazon 商品情報 [online], 13 July 2015 [retrieved: 27 January 2020] Internet: <URL:https://www.amazon.co.jp/%E3%81%93%E3%81%A0%E3%81%BE%E9%A3%9F%E5%93%81-%E6%9C%AC%E6%A0%BC%E4%B9%BE%E7%87%A5%E9%87%8E%E8%8F%9C%E3%83%91%E3%82%A6%E3%83%80%E3%83%BC-%E8%B5%A4%E3%81%97%E3%81%9D%E3%83%91%E3%82%A6%E3%83%80%E3%83%BC%EF%BC%88%E6%9C%89%E5%A1%>, entire text, non-official translation (Kodama Foods, Genuine Dried Vegetable Powder: Red Shiso Powder (Salted). Amazon Product Information.) | 1-7 |
| A | 新素材&新技術情報 肉を軟らかくする梅酢調味料 梅ソフト, フードケミカル, 2008, vol. 24, no. 8, p. 81, left column, non-official translation (New Material & New Technology Information. Ume Soft, the Plum Vinegar Condiment that Softens Meat. Food Chemical.) | 1-7 |
| Y | 永田千夏ほか, 赤紫蘇加工におけるロスマリン酸の変動とその消化管吸収動態, 日本食品科学工学会大会第 52 回大会講演集, 2005, vol. 52, p. 62, 2Ba12 section, non-official translation (NAGATA, Chinatsu et al. Rosmarinic Acid Variation in Red Shiso Processing and its Digestive Tract Absorption Kinetics. Proceedings of the 52nd Annual Meeting of the Japanese Society for Food science and Technology.) | 1-7 |
| Y | YEMENUIGLU, Ahmet et al. Some Characteristics of Polyphenol Oxidase and Peroxidase from Taro (Colocasia antiquorum), Tr. J. of Agriculture and Forestry, 1999, 23, 425-430, table 3 | 1-7 |
| Y | DEMIR, Yavuz et al., Effects of NaCl and Proline on Polyphenol Oxidase Activity in Bean Seedlings, Biologic Plantarum, 2001, 44(4), 607-609, fig. 1, 2 | 1-7 |
| Y | ZORIC, Zoran et al., Stability of Rosmarinic Acid in Aqueous Extracts from Different Lamiaceae Species after in vitro Digestion with Human Gastrointestinal Enzymes, Food Technol. Biotechnol., 2016, 54(1), 97-102, table 1 | 1-7 |

Form PCT/ISA/210 (continuation of second sheet) (January 2015)

**INTERNATIONAL SEARCH REPORT**
Information on patent family members

International application No.

PCT/JP2019/046059

| Patent Documents referred to in the Report | Publication Date | Patent Family | Publication Date |
|---|---|---|---|
| WO 2002/062365 A1 | 15.08.2002 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200262365 A
- JP 2003180286 A
- JP 7187989 A

**Non-patent literature cited in the description**

- **KIDO HIROTSUGU.** *Oleo Science,* 2004, vol. 4 (10), 409-415